(19) Europäisches Patentamt
European Patent Office
Office européen des brevets

(11) Publication number: **0 159 112**
**A1**

(12) # EUROPEAN PATENT APPLICATION

(21) Application number: 85301112.0

(22) Date of filing: 20.02.85

(51) Int. Cl.[4]: C 07 D 213/73
//C07D213/77

(30) Priority: 21.02.84 US 582197

(43) Date of publication of application:
23.10.85 Bulletin 85/43

(84) Designated Contracting States:
AT BE CH DE FR GB IT LI NL SE

(71) Applicant: ELI LILLY AND COMPANY
Lilly Corporate Center
Indianapolis Indiana 46285(US)

(72) Inventor: Campbell, Jack Beuford
1809 South Chester
Indianapolis Indiana 46203(US)

(72) Inventor: Lavagnino, Edward Ralph
4010 Rommel Drive
Indianapolis, Indiana 46208(US)

(72) Inventor: Pike, Andrew Joseph
5226 Cornelius Avenue
Indianapolis Indiana 46208(US)

(74) Representative: Tapping, Kenneth George et al,
Lilly Industries Limited Patent Department Erl Wood Manor
Windlesham Surrey, GU20 6PH(GB)

(54) **Process for the preparation of diaminopyridines.**

(57) A process for preparing diaminopyridines in high yield and in pure form by treating a solution of a nitrohydrazinopyridine with an activated nickel catalyst in the presence of hydrogen gas.

EP 0 159 112 A1

# PROCESS FOR THE PREPARATION OF DIAMINOPYRIDINES

The present invention relates to a new and particularly efficient process for preparing a diaminopyridine of the formula

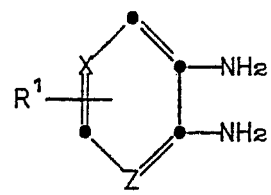

or a salt thereof, wherein $R^1$ is hydrogen or $C_1$-$C_4$ alkyl and one of X and Z is CH and the other is N, comprising treating a nitrohydrazinopyridine of the formula

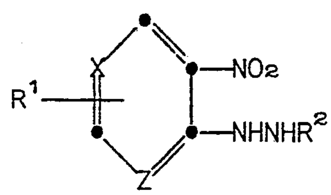

or a salt thereof, wherein $R^2$ is hydrogen, phenyl,

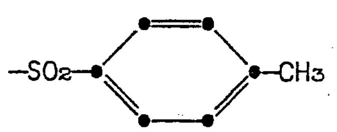 , 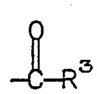

or

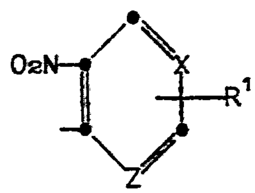 ,

and $R^3$ is $C_1$-$C_4$ alkyl, $C_1$-$C_4$ alkoxy or phenyl, with the proviso that both Z groups and $R^1$ groups are the same, in a suitable solvent with an activated nickel catalyst in the presence of hydrogen gas.

The present process has been found useful in producing large quantities of either a 2,3- or 3,4-diaminopyridine derivative. The process produces the desired product in very high yields and provides the product consistently in high purity, so that the compound may be used in the preparation of biologically active compounds without additional expensive purification steps.

In the above formula, $C_1$-$C_4$ alkyl represents a straight or branched alkyl chain having from one to four carbon atoms. Typical $C_1$-$C_4$ alkyl groups include methyl, ethyl, n-propyl, isopropyl, n-butyl, isobutyl, sec.-butyl, and t-butyl.

The term $C_1$-$C_4$ alkoxy represents a straight or branched alkoxy chain having from one to four carbon atoms. Typical $C_1$-$C_4$ alkoxy groups include methoxy, ethoxy, n-propoxy, sec.-butoxy and the like.

While the entire scope of process variables taught herein are believed operable, the present process does have preferred aspects. Preferably, both $R^1$ and

$R^2$ are hydrogen and Z is CH. Other preferred process conditions will be noted hereinafter.

The process of the present invention is typically conducted as follows. The nitrohydrazinopyridine, or a salt thereof, is dissolved in a suitable solvent, for example any one of the several non-reactive monohydric alcohols such as methanol, ethanol and the like; ethers such as diethyl ether or tetrahydrofuran; and saturated or aromatic hydrocarbons such as pentane, hexane, benzene, toluene, the xylenes and the like. Of these, ethanol is preferred. The concentration of the starting pyridine in the solvent is not critical, but it is preferred to use enough solvent to keep the starting material in solution. A slight excess of solvent is preferably used. Large volumes of solvent are not necessary nor desirable in this process. Typical salts comprehended herein include acid addition salts, such as hydrobromide and especially hydrochloride salts.

An activated nickel catalyst is also employed in the process of the present invention. Raney Nickel is an example of an activated nickel catalyst. This material is widely available commercially or can be prepared by fusing 50 parts nickel with 50 parts aluminum, pulverizing the alloy thus formed, and dissolving out most of the aluminum with sodium hydroxide. Raney Nickel contains hydrogen which dissipates upon storage thereby inactivating the catalyst. Therefore the use of fresh catalyst in the present reaction is preferred, that is, catalyst that is not older than approximately six months. The catalyst is preferably stored under alcohol although other solvents also work. The activated nickel catalyst is typically present in the reaction mixture at a concentration of from about one-tenth part to about 100 parts by weight of starting material to one part of catalyst by weight. It should be noted that greater or lesser quantities of catalyst may be employed if desired depending on the specified conditions.

The process of the present invention is conducted in the presence of hydrogen generally at a pressure in the range of from about standard pressure to approximately 100 pounds per square inch. The reaction is substantially complete after about 1 to 24 hours when conducted at a temperature in the range of about 15°C to about 100°C, more preferably from about 20°C to about 50°C.

Once the process of the present invention is complete, the product may be isolated according to standard procedures. Typically the reaction mixture is filtered through infusorial earth to remove the activated nickel catalyst. The filtrate is then normally concentrated under reduced pressure to provide the appropriate diaminopyridine derivative, or a salt thereof as defined above. The product thus isolated may then be further purified if desired by employing any one of several common techniques such as crystallization from common solvents or column chromatography over solid supports such as silica gel or alumina.

The diaminopyridine compounds prepared by the process of the present invention are useful as intermediates for the preparation of a variety of compounds, for example pharmaceuticals useful for the treatment of

a variety of human disorders. See, e.g. U.S. Patent Nos. 3,985,891 and 4,327,100 (use of 2,3-diaminopyridine in the preparation of imidazo[4,5-b]pyridine inotropic agents); and European Patent Application 72,926 (use of 3,4-diaminopyridine in the preparation of imidazo-[4,5-c]pyridine inotropic agents). The reader is also referred to U.S. Patent No. 4,386,095 which teaches the use of diaminopyridines to ameliorate decreased cognition occurring with ageing in mammals.

The nitrohydrazinopyridine starting materials are easily prepared by a variety of known reactions from assorted readily available starting materials. Certain of these reactions are defined by the scheme that follows:

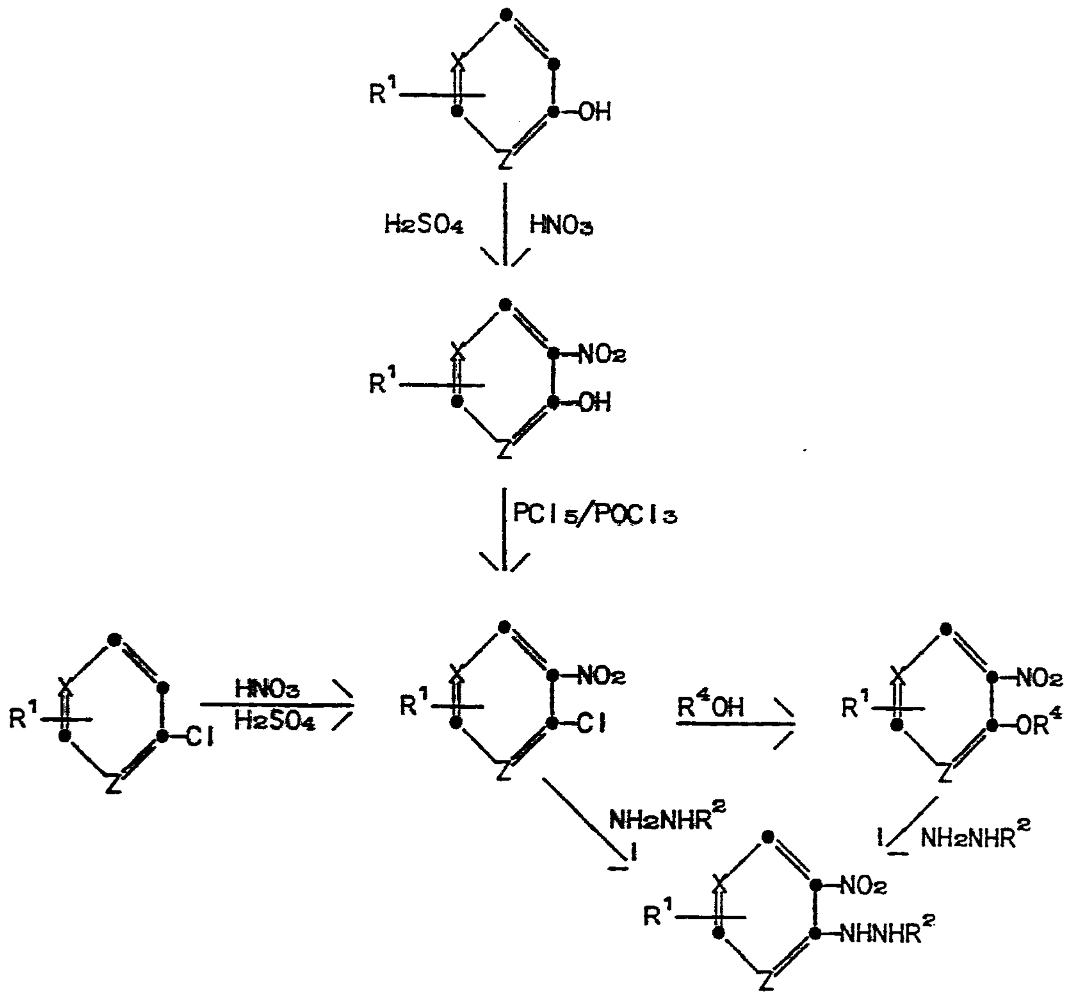


wherein $R^1$ and $R^2$ are as defined above and $R^4$ is $C_1$-$C_4$ alkyl.

The hydroxypyridine and chloropyridine starting materials are generally commercially available or readily prepared by well known procedures. The processes for converting those compounds to the corresponding nitropyridine derivatives with nitric acid and sulfuric acids are reported in the literature as well. See, e.g. Koenigs and Freter, Ber. 57, 1187 (1924). Converting the hydroxynitropyridine to the chloronitropyridine is also readily accomplished by reacting the compound with either phosphorus pentachloride or phosphorus oxychloride. See Koenings et al., supra.

The nitrohydrazinopyridine may be prepared from the corresponding nitrochloro- or nitroalkoxypyridine with a hydrazine or hydrazide derivative according to well known procedures. These processes comprise reacting one mole equivalent of the pyridine starting material with at least one mole equivalent of an appropriate hydrazine or hydrazide derivative in a suitable solvent. Suitable solvents include the polar, aprotic solvents such as dimethylsulfoxide, dimethylformamide and especially acetonitrile or methylene chloride. The reaction is substantially complete after about 1 to 24 hours when conducted at a temperature in the range of from about 25°C to about 200°C, more preferably at the reflux temperature of the reaction mixture. The product is then typically isolated by either collecting the precipitated solid by filtration or concentrating the reaction mixture under vacuum. The product thus isolated may be further purified if desired by standard techniques. Koenigs et al., supra. generally describe the preparation of certain of the

nitrohydrazinopyridine compounds employed herein, or preparations analogous thereto.

The process of the present invention is further illustrated by the following Examples. The Examples are not intended to be limiting in any respect and should not be so construed.

## Example 1

### 2,3-Diaminopyridine

A. 2-Hydrazino-3-nitropyridine

A 5 l. 3-neck round bottom flask with a mechanical stirrer, reflux condenser and thermometer was charged with 100 g (0.63 mol) of 2-chloro-3-nitropyridine and 2.0 l. of acetonitrile. To this solution was added 75 g (2.34 mol) of hydrazine whereupon the color of the reaction mixture changed from yellow to red. As the temperature of the reaction mixture rose to 50°C, orange needles began to precipitate out of solution. When the exotherm had subsided the reaction mixture was heated at about 65°C for 30 minutes whereupon, the crystals went back into solution. The mixture was allowed to cool to room temperature and the precipitated solid was collected by filtration. A portion of the solvent was removed from the mother liquor by evaporation under reduced pressure, whereupon an additional precipitate formed. The solid was collected by filtration. A portion of the collected solids was dissolved in 1N hydrochloric acid and treated with concentrated ammonium hydroxide to provide 2-hydrazino-3-nitropyridine in pure form. mp = 160°-162°C. m/e 154.

B. A solution of 10.0 g of 2-hydrazino-3-nitropyridine, 88 ml of ethanol and 2.0 g of Raney Nickel was hydrogenated at room temperature for five hours at an initial hydrogen pressure of 60 pounds per square inch. The reaction mixture was filtered through Celite and the filtrate was evaporated to dryness under reduced pressure. The residue was recrystallized from benzene to afford 2,3-diaminopyridine. mp = 115°-116°C.

Analysis calculated for $C_5H_7N_3$

Theory: C, 55.03; H, 6.47; N, 38.50;

Found: C, 54.88; H, 6.58; N, 38.48.

Example 2

3,4-Diaminopyridine

A. 3-Nitro-4-hydrazinopyridine

A 12 l. 3-neck round bottom flask fitted with a mechanical stirrer, reflux condenser and heating mantle was charged with 310 g (1.845 mol) of 3-nitro-4-ethoxypyridine and approximately 6 l. of acetonitrile. To this solution was added 70 g (2.19 mol) of hydrazine whereupon the color of the reaction mixture changed from yellow to red. The reaction mixture was refluxed for 4½ hours and allowed to cool to room temperature gradually. The precipitated red crystals were collected by filtration and washed with acetonitrile. The filtrate was chilled with an ice/ethanol bath and the resulting precipitate was collected. The collected solids were combined after being dried at 55°C under vacuum to provide 254.5 g (89% yield) of 3-nitro-4-hydrazinopyridine. mp = 205°-206°C decomposed. m/e 154.

Analysis calculated for $C_5H_6N_4O_2$

Theory: C, 38.96; H, 3.92; N, 36.35;

Found: C, 39.00; H, 3.80; N, 36.13.

B. A solution of 100 g (0.65 mol) of 3-nitro-4-hydrazinopyridine, 3.9 l. of ethanol and 12.0 g of Raney Nickel was hydrogenated at room temperature for 12 hours at an initial hydrogen pressure of 60 pounds per square inch. An uptake of 2.6 moles of hydrogen was accomplished by the reaction. The mixture was filtered through Super-Cel to remove the catalyst and the filtrate was concentrated under reduced pressure. The residue was recrystallized from methanol to provide 63.9 g of 3,4-diaminopyridine. Yield 90%. mp = 200°-205°C. m/e 109.

Analysis calculated for $C_5H_7N_3$

Theory: C, 55.03; H, 6.47; N, 38.50;

Found: C, 55.24; H, 6.70; N, 38.30.

Example 3

3,4-Diaminopyridine monohydrochloride

A. Benzoic acid, 2-(3-nitro-4-pyridinyl)hydrazide monohydrochloride

A solution of 3.2 g (0.02 mol) of 3-nitro-4-chloropyridine and 3.0 g (0.022 mol) of benzoylhydrazine (Aldrich Chemical Co., Milwaukee, Wisconsin) in 100 ml

of methylene chloride was stirred for four hours. A heat lamp was used to provide moderate warming. The precipitated solid was collected by filtration and dried to provide 800 mg of benzoic acid, 2-(3-nitro-4-pyridinyl)hydrazide monohydrochloride following recrystallization from methanol/diethyl ether. mp = 248°-250°C.

Analysis calculated for $C_{12}H_{11}ClN_4O_3$

Theory: C, 48.91; H, 3.76; N, 19.01;

Found: C, 48.77; H, 3.54; N, 18.81.

B. A mixture of 0.8 g (2.7 mmol) of benzoic acid, 2-(3-nitro-4-pyridinyl)hydrazide monohydrochloride and 2.0 g of Raney Nickel in 100 ml of ethanol was hydrogenated at 60°C overnight. The initial hydrogen pressure was 60 psi. The reaction mixture was filtered through Celite and the filtrate was evaporated under reduced pressure. The residue was slurried in ether and the ether decanted. The resulting solid (200 mg) was collected and the structure of the product was verified by thin layer chromatography in 10:10:1 ethyl acetate: ethanol:triethylamine solvent system. m/e 109.

## Example 4

### 2,3-Diaminopyridine

A. 2,2'-Hydrazobis(3-nitropyridine)

A 1 liter 3-neck round bottom flask fitted with a reflux condenser, mechanical stirrer, thermometer

and heating mantle was charged with 7.7 g (0.05 mol) of 2-hydrazino-3-nitropyridine, 7.9 g (0.05 mol) of 2-chloro-3-nitropyridine and 600 ml of acetonitrile. The reaction mixture was refluxed for approximately 24 hours and a small amount of potassium carbonate was added. The mixture was refluxed for another 15 minutes and cooled to about 20°C. The precipitated solid was collected by filtration and the filtrate was refluxed for 24 hours after adding an additional amount of 2-chloro-3-nitropyridine. The mixture was cooled and the solid precipitate collected. The two collected solids were combined, washed with 1N hydrochloric acid and dried in vacuo at 60°C to afford 7.0 g of 2,2'-hydrazobis(3-nitropyridine). mp = 283°-285°C (dec.) Yield 51%.

Analysis calculated for $C_{10}H_8N_6O_4$

Theory: C, 43.48; H, 2.92; N, 30.43;

Found: C, 43.43; H, 2.90; N, 30.34.

B. A mixture of 5.0 g (0.018 mol) of 2,2'-hydrazobis(3-nitropyridine) and 2.0 g of Raney Nickel in 993 ml of ethanol was hydrogenated at room temperature. The initial hydrogen pressure was 60 psi and after approximately 18 hours 100% uptake was observed. The mixture was filtered through Celite and the filtrate was evaporated to dryness under reduced pressure to provide 2,3-diaminopyridine. m/e 109.

## Example 5

### 3,4-Diaminopyridine monohydrochloride

A. 2-(3-Nitro-4-pyridinyl)hydrazinecarboxylic acid, ethyl ester monohydrochloride

A solution of 3.2 g (0.02 mol) of 3-nitro-4-chloropyridine and 2.1 g (0.02 mol) of ethyl carbazate (Aldrich) in 100 ml of methylene chloride was mixed under slight warming. The reaction mixture was stirred at room temperature overnight. The reaction mixture was concentrated under reduced pressure and the resulting residue was recrystallized from methanol/diethyl ether to afford 2.6 g of 2-(3-nitro-4-pyridinyl)hydrazine-carboxylic acid, ethyl ester monohydrochloride. mp = 203°-205°C.

Analysis calculated for $C_8H_{11}ClN_4O_4$

Theory: C, 36.58; H, 4.22; N, 21.33;

Found: C, 36.34; H, 4.24; N, 21.25.

B. A mixture of 1.5 g (5.7 mmol) 2-(3-nitro-4-pyridinyl)hydrazinecarboxylic acid, ethyl ester mono-hydrochoride and 3.0 g of Raney Nickel in 100 ml of ethanol was hydrogenated at an initial pressure of 60 psi at 50°-60°C overnight. The reaction mixture was filtered through Celite and evaporated to dryness under reduced pressure to provide 0.5 g of 3,4-diaminopyridine monohydrochloride. Yield 60% m/e 109.

Example 6

3,4-Diaminopyridine monohydrochloride

A. 4-Methylbenzenesulfonic acid, 2-(3-nitro-4-pyridinyl)-hydrazide monohydrochloride

A solution of 3.2 g (0.02 mol) of 3-nitro-4-chloropyridine and 3.7 g (0.02 mol) of p-toluenesulfonhydrazide (Aldrich) in 150 ml of methylene chloride was stirred overnight at room temperature. The precipitated solid was collected by filtration to afford 2.9 g of crude product. This solid was recrystallized from methanol/diethyl ether to provide 1.4 g of 4-methylbenzenesulfonic acid, 2-(3-nitro-4-pyridinyl)hydrazide monohydrochloride. mp = 206°-208°C dec. m/e 308.

Analysis calculated for $C_{12}H_{13}N_4O_4SCl$

Theory: C, 41.80; H, 3.80; N, 16.25;

Found: C, 41.69; H, 3.58; N, 16.47.

B. A mixture of 1.4 g (0.004 mol) of 4-methylbenzenesulfonic acid, 2-(3-nitro-4-pyridinyl)hydrazide monohydrochloride and 2.0 g of Raney Nickel in 100 ml of ethanol was hydrogenated at 60°C and under 60 psi of hydrogen pressure for approximately 18 hours. The reaction mixture was filtered through Celite. The filtrate was evaporated to dryness under reduced pressure to afford 600 mg of a solid. Thin layer chromatography of the solid with a 10:10:1 ethyl acetate:ethanol:triethylamine solvent system indicated that 3,4-diaminopyridine was present when compared with an authentic reference sample.

## Example 7

### 3,4-Diaminopyridine monohydrochloride

A. Acetic acid, 2-(3-nitro-4-pyridinyl)hydrazide monohydrochloride

A solution of 3.2 g (0.02 mol) of 3-nitro-4-chloropyridine and 1.5 g (0.02 mol) of acethydrazide (Aldrich) in 100 ml of methylene chloride was stirred at room temperature overnight. The mixture was concentrated under vacuum to provide a gummy solid. The solid was triturated in methanol and the precipitated solid was collected by filtration to afford 2.5 g of acetic acid, 2-(3-nitro-4-pyridinyl)hydrazide monohydrochloride. The solid was recrystallized from methanol/diethyl ether to afford 2.1 g of product. mp = 220°-222°C. m/e 196. The structure of the product was also verified by NMR.

B. A mixture of 2.0 g (0.009 mol) of acetic acid, 2-(3-nitro-4-pyridinyl)hydrazide monohydrochloride and 5.0 g of Raney Nickel in 100 ml of ethanol was hydrogenated at 60°C and under 60 psi of hydrogen pressure for approximately 18 hours. The reaction mixture was filtered through Celite and the filtrate was evaporated to dryness under reduced pressure. The residue existed as a gummy solid and a mass spectrograph of the solid indicated the presence of 3,4-diaminopyridine.

CLAIMS

1. A process for preparing a diaminopyridine of the formula

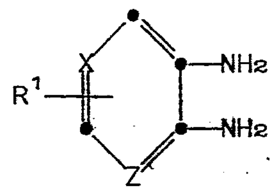

or a salt thereof, wherein $R^1$ is hydrogen or $C_1$-$C_4$ alkyl and one of X and Z is CH and the other is N, comprising treating a nitrohydrazinopyridine of the formula

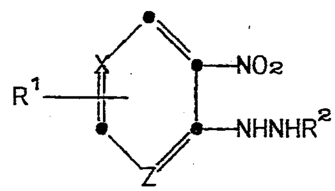

or a salt thereof, wherein $R^2$ is hydrogen, phenyl,

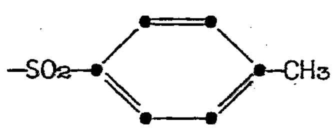 , 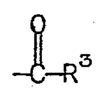

or

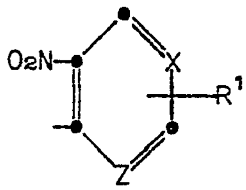

,

and $R^3$ is $C_1$-$C_4$ alkyl, $C_1$-$C_4$ alkoxy or phenyl, with the proviso that both Z groups and $R^1$ groups are the same, in a suitable solvent with an activated nickel catalyst in the presence of hydrogen gas.

2. A process of Claim 1 wherein $R^1$ is hydrogen.

3. A process of Claim 1 wherein Z is N.

4. A process of Claim 1 wherein Z is CH.

5. A process of Claim 3 or 4 wherein $R^2$ is hydrogen.

6. A process of Claim 3 or 4 wherein $R^2$ is

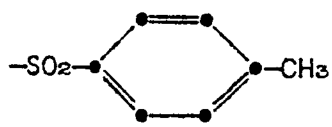

7. A process of Claim 3 or 4 wherein $R^2$ is $-\overset{O}{\overset{\|}{C}}R^3$ and $R^3$ is $C_1$-$C_4$ alkyl.

8. A process of Claim 3 or 4 wherein $R^2$ is $-\overset{O}{\overset{\|}{C}}R^3$ and $R^3$ is $C_1$-$C_4$ alkoxy.

9. A process of Claim 3 or 4 wherein $R^2$ is $-\overset{O}{\overset{\|}{C}}R^3$ and $R^3$ is phenyl.

10. A process of Claim 3 or 4 wherein $R^2$ is

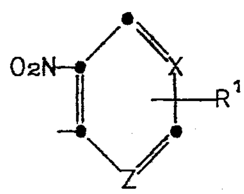

0159112

European Patent Office

Application number

EP 85 30 1112

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | J. HETEROCYCLIC CHEMISTRY, vol. 8, no. 1, February 1971; A. LEWIS et al. "Synthesis and Spectral Data of Pyrido [3,2-e]-as-triazines", pages 41-46<br>* Page 41, right hand column, lines 27-32, experimental; page 43, compound IIIa; page 46, compound IVa * | 1-3,5, 7 | C 07 D 213/73 //<br>C 07 D 213/77 |
| Y | idem<br>--- | 1-5,7, 9 | |
| Y | US-A-3 549 631 (LEWIS et al.)<br>* Column 3, lines 31-36, 59-65; column 1, lines 39-41; examples 7,13 *<br>--- | 1-3,5, 7 | TECHNICAL FIELDS SEARCHED (Int. Cl.4) |
| Y | GB-A-1 492 073 (EGYT GYOGYSZERVE-GYESZETI GYAR)<br>* Page 1, lines 37, 52-61; page 2, lines 56-59; example 15 *<br>--- -/- | 1-3,9 | C 07 D 213/00 |

The present search report has been drawn up for all claims

| Place of search | Date of completion of the search | Examiner |
|---|---|---|
| BERLIN | 17-05-1985 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

European Patent Office

# EUROPEAN SEARCH REPORT

0159112
Application number

EP 85 30 1112



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | J. HETEROCYCLIC CHEMISTRY, vol. 8, no. 1, February 1971; A. LEWIS et al. "Cyclizations Leading to Pyrido [3,4-e]-and [4,3-e]-as-triazines. Ring Interconversion of a Pyrido-as-triazine 1-Oxide to a Pyridotriazole", pages 47-49 * Experimental, page 49, compounds II (R=H), III (R=COCH3) * --- | 1,2,4, 5,7 | |
| Y | US-A-3 597 427 (LEWIS et al.) * Column 1, lines 35-38; column 2, line 71 column 3, line 4; column 3, lines 27-33; examples 2,4 * --- | 1,2,4, 5,7 | |
| Y | GB-A-1 483 025 (EGYT GYOGYSZERVE-GYESZETI GYAR) * Page 1, lines 44,60 - page 2, line 7 * --- | 1,2,4, 9 | TECHNICAL FIELDS SEARCHED (Int. Cl.4) |
| Y | DE-A-2 427 377 (MORTON-NORWICH INC.) * Example C * --- | 1,2,4, 5 | |
| A | FR-A-2 479 224 (STERLING DRUG INC.) * Page 9, lines 21-26; example F1 * --- -/- | 1 | |

The present search report has been drawn up for all claims

| Place of search | Date of completion of the search | Examiner |
|---|---|---|
| BERLIN | 17-05-1985 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0159112

Application number

EP 85 30 1112

Page 3

## DOCUMENTS CONSIDERED TO BE RELEVANT

| Category | Citation of document with indication where appropriate, of relevant passages | Relevant to claim | CLASSIFICATION OF THE APPLICATION (Int Cl 4) |
|---|---|---|---|
| A | CHEMICAL ABSTRACTS, vol. 57, no. 6, 17th September 1962, Columbus, Ohio, USA; T. UEDA et al. "Reduction of arysulfonylhydrazines with activated Raney nickel", page 7151, left hand column e, line 8 & CHEM. PHARM. BULL (TOKYO), no. 9, 1961, pages 71 -72<br>--- | 1,6 | |
| A | HOUBEN-WEYL "Methoden der organischen Chemie" 4th edition 1957, GEORG THIEME VERLAG, Stuttgart R. SCHROETER "Amine durch Reduktion", pages 531-538 * Page 538, lines 25-28 *<br>--- | 1,10 | |
| A | CHEMISCHES ZENTRALBLATT, vol. 136, no. 44, 27th Ocotber 1965, Berlin; J.B. POLYA et al. "Polyazanaphthaline. 1. Mitt. Synthese von Pyrimido-[5.4-e]-asymm.-Triazinen ", page 13893, abstract no. 1014 * right hand column, lines 73-75, 34-38 *<br>----- | 1,2,3, 9 | TECHNICAL FIELDS SEARCHED (Int. Cl.4) |

The present search report has been drawn up for all claims

| Place of search | Date of completion of the search | Examiner |
|---|---|---|
| BERLIN | 17-05-1985 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82